# FASCICULE DE BREVET EUROPEEN

(11) **EP 0 716 620 B1**
(45) Date de publication et mention de la délivrance du brevet: **09.04.1997**
(21) Numéro de dépôt: 94923772.1
(22) Date de dépôt: 03.08.1994
(51) Int. Cl.: A61N 1/30

(54) **RESERVOIR IMPREGNABLE D'UNE SOLUTION DE PRINCIPE ACTIF, POUR DISPOSITIF IONOPHORETIQUE D'ADMINISTRATION TRANSDERMIQUE DE MEDICAMENTS, ET PROCEDE DE FABRICATION D'UN TEL RESERVOIR**
BEHÄLTER, DER MIT EINER LÖSUNG EINES AKTIVEN PRINZIPES IMPRÄGNIERBAR IST, FÜR EINE IONTOPHORETISCHE VORRICHTUNG ZUR TRANSDERMALEN VERABREICHUNG VON MEDIKAMENTEN, UND VERFAHREN ZUR HERSTELLUNG EINES SOLCHEN BEHÄLTERS
RESERVOIR IMPREGNABLE WITH AN ACTIVE SOLUTION FOR AN IONOPHORETIC TRANSDERMIC DRUG DELIVERY DEVICE AND METHOD OF MANUFACTURE OF SUCH A RESERVOIR

(30) Priorité: 30.08.1993 FR 9310360
(43) Date de publication de la demande: 19.06.1996
(73) Titulaire: LABORATOIRES D'HYGIENE ET DE DIETETIQUE L.H.D., 75008 Paris (FR)
(72) Inventeur: DHUIQUE-MAYER, Daniel, F-21000 Dijon (FR); LIORZOU, Laurent, 31200 TOULOUSE (FR)
(74) Mandataire: Colas, Jean-Pierre
(86) Numéro de dépôt international: FR9400974
(87) Numéro de publication internationale: WO9506496

(56) Documents cités:
- EP-A- 0 409 067
- WO-A-92/10235
- US-A- 5 069 908

## Description

La présente invention est relative à un réservoir imprégnable d'une solution de principe actif pour être utilisé dans un dispositif ionophorétique d'administration transdermique de médicaments, et, plus particulièrement, à un tel réservoir chargé d'un matériau divisé imprégnable avec cette solution. La présente invention a aussi pour but de fournir un procédé de fabrication d'un tel réservoir.

L'administration transdermique de médicaments assistée par ionophorèse est une technique aujourd'hui bien connue, qui a fait l'objet de nombreuses publications telles que, par exemple, la demande de brevet européen No. 409067 et la demande internationale de brevet WO 92/10235 auxquelles on pourra se référer pour plus de détail concernant les caractéristiques essentielles de cette technique médicale.

Celle-ci consiste à appliquer contre la peau d'un patient un réservoir d'un principe actif en solution et à forcer le passage à travers la peau d'une forme ionisée de ce principe actif, par l'action d'un champ électrique convenablement orienté. Pour ce faire une électrode plane est plaquée sur le réservoir et une différence de potentiel électrique est établie entre cette électrode et une électrode voisine, éventuellement plaquée directement sur la peau du patient. L'électrode plaquée sur le réservoir est polarisée de manière à repousser les ions du principe actif sous la peau du patient pour qu'ils passent dans l'appareil circulatoire de celui-ci.

Dans l'état actuel de la technique, le réservoir est couramment constitué d'un hydrogel susceptible d'être imprégné de la solution de principe actif. Il n'est pas chargé en permanence de cette solution. On le conserve en effet à l'état "sec", pour éviter des problèmes de stabilité, par exemple ceux résultant de contamination et/ou d'incompatibilité de matériaux, et on l'imbibe de la solution immédiatement avant l'emploi.

Pour la commodité de cet emploi, il est alors nécessaire que l'imprégnation de l'hydrogel contenu dans le réservoir s'effectue rapidement, en un temps de l'ordre de la minute par exemple. L'imprégnation de l'hydrogel doit aussi être homogène et complète.

La présente invention a précisément pour but de réaliser un tel réservoir, à chargement rapide, complet et homogène.

La présente invention a aussi pour but de fournir un procédé de fabrication d'un tel réservoir qui soit exploitable industriellement, c'est-à-dire un procédé capable de fournir un volume de production important à un prix de revient de fabrication aussi contenu que possible.

On atteint ces buts de l'invention, ainsi que d'autres qui apparaîtront à la lecture de la description qui va suivre, avec un réservoir chargé d'un matériau divisé imprégnable avec une solution de principe actif, remarquable en ce qu'il comprend a) une feuille de support en un matériau lacunaire perméable à la solution et propre à contribuer à la tenue mécanique du réservoir, b) une couche de particules du matériau imprégnable et c) une couche de substratage interposée entre une face de la feuille de support et la couche de particules pour retenir ces dernières sur la feuille, cette couche de substratage étant constituée d'une substance miscible avec la solution de principe actif.

L'utilisation d'un matériau particulaire imprégnable permet de disposer d'une grande surface d'absorption de la solution de principe actif, ce qui accélère l'imprégnation du réservoir. L'épaisseur de la couche de particules est, de préférence, de l'ordre de grandeur de la dimension moyenne de celles-ci ce qui assure un étalement rapide et homogène de la solution dans le réservoir. La miscibilité de la couche de substratage avec la solution de principe actif qui devra imprégner le réservoir assure la continuité électrique entre ce réservoir et une électrode conçue pour le recevoir.

Pour être fixé sur une telle électrode appartenant à un dispositif ionophorétique d'administration transdermique de médicaments, le réservoir peut être garni d'une feuille adhésive double face accolée à la feuille de support sur la face de celle-ci qui est opposée à celle enduite de la couche de substratage, cette feuille adhésive étant régulièrement ajourée pour assurer un contact électrique entre la solution et l'électrode.

Suivant l'invention, la couche de substratage du réservoir est constituée, de préférence, par l'une des substances du groupe formé par la glycérine, la diacétine, l'homoacétine, le polyéthylène glycol.

Suivant l'invention, encore, le matériau divisé imprégnable qui garnit le réservoir est choisi de préférence parmi les dérivés cellulosiques tels que l'hydroxypropylméthylcellulose, l'hydroxyéthylcellulose, l'hydroxypropylcellulose, la carboxyméthylcellulose, la méthylcellulose, les polymères et copolymères de l'acide acrylique, de l'acide méthacrylique et de leurs esters, les gels de polyalkylène glycol, la polyvinyl pyrrolidone et ses dérivés, l'alcool polyvinylique, le polyacrylamide et dérivés, les gommes naturelles, les dérivés amylacés hydrosolubles et les copolymères (vinyl méthyl éther-anhydride maléique).

L'invention fournit aussi un procédé de fabrication du réservoir suivant la présente invention, remarquable en ce que a) on enduit une face de la feuille de support avec une couche adhésive d'un produit de substratage et b) on poudre la couche ainsi formée avec des particules du matériau imprégnable par la solution de principe actif. La feuille de support peut prendre la forme d'une bande défilant dans un poste d'enduction d'une face de cette bande avec le produit de substratage, puis dans un poste de poudrage de la couche de ce produit formée sur la bande. Suivant un mode de réalisation préféré de la présente invention, le poudrage de la bande s'opère par tamisage des particules du matériau imprégnable au-dessus de la couche adhésive de substratage.

La bande ainsi fabriquée circule à grande vitesse dans une machine de fabrication ce qui assure un grand volume de production à coût réduit. La bande est ensuite automatiquement soudée et tronçonnée et les pièces obtenues constituent des réservoirs suivant l'invention.

D'autres caractéristiques et avantages de la présente invention apparaitront à la lecture de la description qui va suivre et à l'examen du dessin annexé dans lequel :
- la figure 1 est une vue en perspective et en coupe d'un mode de réalisation préféré du réservoir suivant l'invention,
- la figure 2 est une vue agrandie du détail repéré A sur la figure 1, qui fait apparaître les diverses couches de produit constituant le réservoir suivant l'invention, et
- les figures 3, 4 et 5 sont des schémas de divers postes d'une machine conçue pour mettre en oeuvre le procédé de fabrication, suivant l'invention, du réservoir des figures 1 et 2.

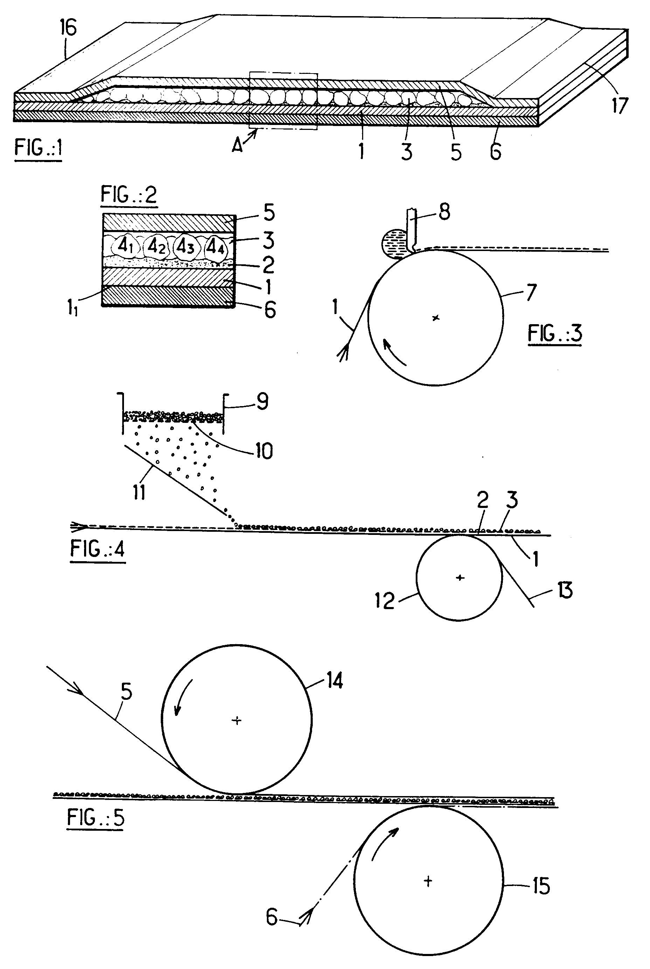

Comme représenté sur la figure 1, le réservoir suivant l'invention prend la forme d'une pièce plate, rectangulaire, typiquement d'une surface de 6 cm x 2 cm, par exemple. Il doit comprendre un matériau imprégnable avec une solution de principe actif et doit être capable de retenir de 1 à 2 ml de cette solution, par exemple.

Pour ce faire, le réservoir comprend essentiellement une feuille de support 1 en un matériau lacunaire, imprégnée d'une couche 2 d'une substance de substratage déposée sur une face de la feuille 1 et présentant une surface propre à retenir par adhérence une couche 3 de particules d'un matériau divisé imprégnable avec une solution de principe actif.

La couche adhésive 2 imprègne elle-même les lacunes de la feuille de support 1, ce qui assure une bonne adhérence de cette couche sur cette feuille. Les particules 4₁,4₂,4₃,4₄, etc... du matériau imprégnable sont régulièrement distribuées sur la couche adhésive 2 et retenues par cette couche dans cette distribution.

La couche 3 offre ainsi une grande surface d'absorption, grâce au cumul des surfaces des particules, lorsqu'on l'imprègne avec une solution de principe actif. L'absorption de cette solution en est fortement accélérée.

L'étalement uniforme de la solution dans la couche est aussi favorisé par la faible épaisseur, de l'ordre de grandeur du diamètre moyen des particules, et la planéité de cette couche.

Suivant un premier mode de réalisation du réservoir suivant l'invention, celui-ci peut être constitué des seules couches 1, 2 et 3.

Cependant, suivant un mode de réalisation préféré, on renforce la cohésion du réservoir par une feuille de couverture 5 qui recouvre la couche des particules 4ᵢ pour assurer que celles-ci restent bien maintenues contre la feuille de support 1.

Le réservoir ainsi constitué doit être fixé, du côté de la feuille de support 1, sur une électrode formée, par exemple, d'une couche conductrice d'argent/chlorure d'argent. Cette fixation s'opère commodément à l'aide d'une feuille adhésive double face initialement solidaire soit du réservoir soit de l'électrode.

Suivant l'invention, on améliore l'adhérence de cette feuille sur le réservoir en plaquant sur la face 1ₗ de la feuille de support 1, une feuille 6 en un matériau ajouré telle qu'une grille à mailles fines, réalisée en un matériau électriquement isolant. La feuille 6 est soudée à la feuille de support au niveau des extrémités de celle-ci voisines de ses bords transversaux 16,17, de même que la feuille de couverture 5. Une face de la feuille adhésive double face (non représentée) est collée contre la grille 6, elle-même plaquée contre la feuille de support 1, l'autre face de la feuille adhésive étant destinée à être appliquée contre la surface de l'électrode.

On remarquera qu'en l'absence de la grille, la feuille adhésive ne pourrait adhérer à la feuille de support, imprégnée de glycérine, par exemple, constituant la substance de substratage, comme on le verra plus loin. La cohésion de l'ensemble du réservoir est assurée par les soudures formées au voisinage des bords 16,17.

La continuité électrique du réservoir, depuis la feuille de couverture 5 jusqu'à l'électrode adhérant à la grille 6, est assurée en utilisant des matériaux lacunaires pour constituer les feuilles 1 et 5 et une couche de substratage 2 miscible avec la solution de principe actif. Cette solution est injectée dans le réservoir à l'aide d'une seringue, par exemple, juste avant l'application de l'ensemble réservoir/électrode sur la peau d'un patient, en vue d'une administration transdermique de médicaments assistée par ionophorèse. La solution imbibe alors les particules 4ᵢ, la couche de substratage 2 et les feuilles lacunaires 1 et 5. La feuille adhésive double face qui fixe le réservoir sur l'électrode peut être par exemple elle-même réalisée avec un support et des produits adhésifs conducteurs de l'électricité. La continuité électrique au niveau de la feuille adhésive peut aussi être obtenue en ajourant ladite feuille de manière que la solution de principe actif puisse venir mouiller l'électrode à travers cette feuille.

Divers matériaux peuvent être utilisés pour constituer les feuilles ou couches 1 à 6. C'est ainsi que, pour les feuilles 1 de support et 5 de couverture (facultative) on propose, suivant l'invention, d'utiliser un matériau tissé ou non-tissé, un feutre, une grille ou une feuille de mousse de matière plastique à pores ouverts, de faible épaisseur. Ce matériau doit être suffisamment fin et ajouré, de grammage préférentiellement compris entre 10 et 50 g/m² et de préférence de l'ordre de 15 g/m², pour qu'un gel, formé à partir des particules 4ᵢ imprégnées de la solution de principe actif, puisse le traverser. Les substances constituant les feuilles 1 et 5 sont de préférence des polymères : polyéthylène, polypropylène, polyamide, polyester, polyuréthanne, cellulose, viscose, copolymère oléfinique, polychlorure de vinyle, polymère cellulosique, acrylique ou méthacrylique.

La couche de substratage 2 dont on imprègne la feuille de support 1 est constituée par un liquide miscible avec la solution aqueuse de principe actif. Ce liquide doit être suffisamment visqueux pour ne pas couler de la feuille de support 1 qu'il imprègne, et suffisamment adhésif pour retenir les particules 4ᵢ absorbantes déposées sur la surface ainsi imprégnée de cette feuille de support. Sa viscosité est comprise de préférence entre 100 et 1000 cps. On peut utiliser à cet effet de la glycérine, de la diacétine, de l'homoacétine, un polyéthylène glycol (ou polyoxyéthylène) de faible masse moléculaire, ou toute substance présentant des caractéristiques équivalentes que l'homme de métier pourra identifier et choisir à l'aide de ses connaissances normales.

La substance constituant les particules absorbantes 4ᵢ est de préférence choisie parmi les polymères hydrophiles ou hydrosolubles. Elle se présente alors sous forme de poudre, de paillettes ou de fibres. Cette forme divisée favorise la répartition du liquide par capillarité à la surface de l'électrode. Cette substance est choisie parmi les suivantes, prises séparément ou en mélange :
- dérivés cellulosiques tels que hydroxypropyl-méthyl cellulose, hydroxyéthylcellulose, hydroxypropylcellulose, carboxyméthylcellulose, méthylcellulose,
- polymères et copolymères de l'acide acrylique, de l'acide méthacrylique et de leurs esters, super-absorbants,
- polyéthylène glycol, polypropylène glycol,
- polyvinyl pyrrolidone et dérivés,
- alcool polyvinylique,
- polyacrylamide et dérivés,
- gommes naturelles, gomme guar, gomme xanthane, pectine, carraghénane, farine de caroube, polysaccharides en général,
- dérivés amylacés hydrosolubles,
- copolymères (vinyl méthyl éther-anhydride maléique).

Les particules 4ᵢ peuvent aussi être obtenues par broyage d'une couche solide réalisée à partir d'un mélange des produits cités ci-dessus, éventuellement additionné de fibres textiles hydrophiles comme le coton. Cette technique est préférée au dépôt direct sur la feuille de support 1, dûment substratée, d'un mélange de différentes substances et de fibres textiles.

Suivant l'invention, les particules obtenues présentent de préférence un diamètre moyen compris entre 80 µm et 250 µm, elles sont déposées en une fine couche de 300 µm environ avec un grammage de 30 à 250 g/m², sur une couche de substratage d'une épaisseur variant entre 100 et 200 µm.

On a remarqué que le dépôt d'une couche de substratage tel que de la glycérine sur une feuille de support lacunaire en non-tissé par exemple, fait apparaître une certaine irrégularité de la surface de cette couche qui "recopie", en quelque sorte, la surface lacunaire irrégulière sur laquelle elle a été déposée. Pour pallier cet inconvénient, suivant la présente invention, on peut enduire la feuille de non-tissé d'un hydrogel de polyoxyéthylène ou autre substance équivalente, préalablement au dépôt de la couche de glycérine. Pour ce faire, on peut disposer la feuille de support 1 sur une feuille protectrice pelable, telle qu'un papier siliconé, de manière à donner à l'ensemble une tenue mécanique suffisante. On procède ensuite à l'enduction de la feuille par l'une quelconque des techniques d'enduction connues, au rouleau, à la râcle, etc... et on sèche ensuite la feuille de non-tissé ainsi enduite. Après l'enlèvement de la feuille de papier siliconé, il apparaît que les deux faces de la feuille de support présentent une surface très lisse. On peut utiliser à cet effet un hydrogel de polyoxyéthylène d'une masse moléculaire de 300000 vendue par la société belge JANSSEN CHEMICA, en solution à 2%. La surface ainsi lissée du non-tissé peut ensuite recevoir une couche de glycérine d'épaisseur régulière et de bon état de surface.

On se réfère maintenant aux figures 3 à 5 du dessin annexé pour décrire un procédé de fabrication préféré du réservoir suivant la présente invention. Suivant ce procédé, on commence par déposer sur la feuille de support 1 une couche du produit de substratage, de la glycérine par exemple, à raison d'environ 75 g/m². Pour ce faire, on forme une bande du matériau, non-tissé par exemple, constituant la feuille 1 de support et on la double d'une bande conforme d'un papier protecteur siliconé pelable qui renforce sa tenue mécanique. On peut alors faire défiler la bande dans un poste de couchage de la glycérine. On a représenté schématiquement à la figure 3 un tel poste. La bande 1 et son papier siliconé sont entraînés par un rouleau 7 motorisé sous une râcle 8 qui règle l'épaisseur de la couche en retenant un ménisque 9 de glycérine formant réserve, réalimentée par des moyens (non représentes) bien connus de l'homme de l'art.

Bien entendu d'autres techniques d'enduction connues pourraient être utilisées en lieu et place de celles illustrées à la figure 3. C'est ainsi que l'on pourrait procéder par enduction au rouleau de transfert, par calandrage, par foulardage, par pulvérisation, ou par diverses techniques utilisées en impression telles que par exemple la sérigraphie.

La bande enduite de glycérine est entraînée dans un poste de poudrage tel que, par exemple, celui représenté à la figure 4. Celui-ci comprend une trémie 9 dont le fond est fermé par un tamis 10, éventuellement vibrant. Un hydrogel déshydraté en poudre, constituant le matériau imprégnable de solution de principe actif du réservoir selon l'invention, est disposé dans la trémie. La poudre tombe du tamis sur une rampe 11 qui distribue régulièrement la poudre sur la surface de la couche de glycérine formée sur la bande 1 de support qui circule sous cette rampe, grâce à l'entraînement développé par un rouleau motorisé 12. Celui-ci sert aussi à peler la bande 13 de papier siliconé qui supporte jusqu'alors la bande 1. Celle-ci garnie de la couche 3 d'hydrogel en poudre fixée sur la surface de la couche de glycérine, passe ensuite dans le poste représenté à la figure 5 pour être "complexée" avec une bande de couverture 5, au niveau d'un rouleau 14, et une bande de la grille 6, au niveau d'un rouleau 15, dans l'hypothèse où de tels éléments 14,15 sont incorporés au réservoir suivant l'invention.

La liaison des bandes 1,5 et 6 entre elles pourrait être renforcée par diverses techniques telles que la soudure par calandrage à chaud en des points régulièrement distribués sur la surface des bandes accolées, ou leur assemblage par des points de colle distribués de même.

La bande de complexe ainsi formée est finalement thermoscellée ou soudée au niveau des bords transversaux 16 et 17 de chaque réservoir fabriqué (voir figure 1) puis tronçonnée au niveau de ces bords pour individualiser chaque réservoir. On notera qu'il n'est pas nécessaire de souder celui-ci sur ses quatre bords.

Le procédé de fabrication décrit ci-dessus, qui fait appel à une bande défilant en continu, soudée et tronçonnée automatiquement, autorise des cadences de production élevées et donc des coûts de fabrication réduits, conformément aux objectifs annoncés en préambule de la présente description.

Bien entendu l'invention n'est pas limitée aux modes de réalisation décrits et représentés qui n'ont été donnés qu'à titre d'exemple. C'est ainsi que des techniques de poudrage autres que le tamisage pourraient être utilisées, par exemple le soufflage de la poudre par des buses, le passage de la bande enduite du produit à surface adhésive dans un lit fluidisé de poudre, etc .... De même, le réservoir peut former un tout avec l'électrode et l'adhésif double-face être utilisé pour les plaquer l'un sur l'autre.

## Revendications

1. Réservoir imprégnable d'une solution de principe actif pour être utilisé dans un dispositif ionophorétique d'application transdermique de médicaments, ce réservoir, chargé d'un matériau divisé imprégnable avec cette solution, étant caractérisé en ce qu'il comprend :
a) une feuille de support (1) en un matériau lacunaire perméable à la solution et propre à contribuer à la tenue mécanique du réservoir,
b) une couche (3) de particules du matériau imprégnable, et
c) une couche (2) de substratage interposée entre une face de la feuille de support et la couche (3) de particules pour retenir ces dernières sur la feuille de support (1), cette couche (2) de substratage étant constituée d'une substance miscible avec la solution de principe actif.

2. Réservoir conforme à la revendication 1, caractérisé en ce que l'épaisseur de ladite couche (3) de particules est de l'ordre de grandeur de la dimension moyenne de ces particules.

3. Réservoir conforme à l'une quelconque des revendications 1 et 2, caractérisé en ce qu'il est garni d'une feuille adhésive double-face accolée à la feuille de support (1) sur la face de celle-ci qui est opposée à celle enduite de la couche (2) de substratage.

4. Réservoir conforme à la revendication 3, caractérisé en ce que ladite feuille adhésive est collée par son autre face sur une couche métallique constituant une électrode.

5. Réservoir conforme à l'une quelconque des revendications 3 et 4, caractérisé en ce qu'une feuille (6) en un matériau ajouré est interposée entre la feuille de support (1) et la feuille adhésive double-face.

6. Réservoir conforme à l'une quelconque des revendications 1 à 5, caractérisé en ce que la feuille de support (1) en matériau lacunaire est imprégnée d'un hydrogel de polyoxyéthylène, préalablement au dépôt de la couche de substratage.

7. Réservoir conforme à l'une quelconque des revendications 1 à 6, caractérisé en ce qu'il comprend une feuille de couverture (5) en un matériau lacunaire, recouvrant la couche (3) de particules du matériau imprégnable.

8. Réservoir conforme à l'une quelconque des revendications 1 à 7, caractérisé en ce que les diverses feuilles et couches le composant sont conformes et soudées les unes aux autres par des bords.

9. Réservoir conforme à l'une quelconque des revendications 1 à 8, caractérisé en ce que la feuille de support (1) est réalisée en un des produits du groupe constitué par les produits tissés ou non-tissés, un feutre, une grille, une mousse poreuse, en un composé polymère du groupe constitué par le polyéthylène, le polypropylène, les polyamides, les polyesters, le polyuréthanne, la cellulose, la viscose, les copolymères oléfiniques, le polychlorure de vinyle, les polymères cellulosiques, acryliques ou méthacryliques.

10. Réservoir conforme à l'une quelconque des revendications 1 à 9, caractérisé en ce que la couche (2) de substratage est constituée de l'une des substances du groupe constitué par : la glycérine, la diacétine, l'homoacétine, le polyéthylène glycol.

11. Réservoir conforme à l'une quelconque des revendications 1 à 10, caractérisé en ce que le matériau divisé imprégnable est constitué de l'une des substances du groupe formé par : les dérivés cellulosiques tels que l'hydroxypropyl-méthylcellulose, l'hydroxyéthylcellulose, l'hydroxypropylcellulose, la carboxyméthylcellulose, la méthylcellulose, les polymères et copolymères de l'acide acrylique, de l'acide méthacrylique et de leurs esters, les gels de polyalkylène glycol, la polyvinyl pyrrolidone et ses dérivés, l'alcool polyvinylique, le polyacrylamide et dérivés, les gommes naturelles, les dérivés amylacés hydrosolubles et les copolymères (vinyl méthyl éther-anhydride maléique).

12. Réservoir conforme à la revendication 11, caractérisé en ce que le matériau divisé imprégnable est constitué de particules de 80 µm à 250 µm, la couche (3) de ce matériau déposée sur la couche (2) de substratage présentant une épaisseur maximale de 300 µm environ.

13. Réservoir conforme à l'ensemble des revendications 7 et 9, caractérisé en ce que la feuille de couverture (5) et la feuille de support (1) sont constitués en des substances polymères du même groupe.

14. Réservoir conforme à l'une quelconque des revendications 5 à 13, caractérisé en ce que la feuille (6) en matériau ajouré est constituée par une grille, un feutre, un produit tissé ou non-tissé, en matière polymère.

15. Procédé de fabrication d'un réservoir conforme à l'une quelconque des revendications 1 à 14, caractérisé en ce que (a) on enduit une face de la feuille de support (1) avec une couche adhésive (2) d'un produit de substratage et (b) on poudre la couche ainsi formée avec des particules (4ᵢ) du matériau imprégnable par la solution de principe actif.

16. Procédé conforme à la revendication 15, caractérisé en ce que la feuille de support (1) prend la forme d'une bande défilant dans un poste d'enduction (7,8) d'une face de cette bande avec la substance de substratage puis dans un poste de poudrage (9,10,11) de la couche de cette substance formée sur la bande.

17. Procédé conforme à la revendication 16, caractérisé en ce que la bande en défilement est doublée par une bande (13) en un matériau de protection pelable.

18. Procédé conforme à la revendication 17, caractérisé en ce que la bande doublée est imprégnée d'un hydrogel de polyoxyéthylène, puis séchée, avant de passer dans le poste d'enduction (7,8) de la substance de substratage.

19. Procédé conforme à l'une quelconque des revendications 17 et 18, caractérisé en ce que, à la sortie du poste de poudrage (9,10,11), la bande de protection (13) est pelée et la bande (1,2,3) substratée et poudrée est ensuite pincée entre une bande de couverture (5) en un matériau ajouré recouvrant la couche (3) de poudre et une bande de grille (6) appliquée contre l'autre face de la bande substratée et poudrée (1,2,3).

20. Procédé conforme à la revendication 19, caractérisé en ce que le produit en bande obtenu est soudé régulièrement aux dimensions finales d'un réservoir puis découpé suivant ces soudures (16,17) pour individualiser chaque réservoir.

21. Procédé conforme à la revendication 19, caractérisé en ce que le produit en bande obtenu est calandré a chaud pour renforcer sa cohésion.

22. Procédé conforme à l'une quelconque des revendications 15 à 21, caractérisé en ce qu'on tamise les particules du matériau imprégnable pour former une couche de telles particules sur la couche adhésive de substratage (2).

## Claims

1. Reservoir which can be impregnated with a solution of active principle in order to be used in an iontophoretic device for transdermal delivery of medicinal products, this reservoir, loaded with a divided material which can be impregnated with this solution, being characterized in that it comprises:
a) a support sheet (1) made of a cavitied material which is permeable to the solution and capable of contributing to the mechanical strength of the reservoir,
b) a layer (3) of particles of the impregnatable material, and
c) a substrative layer (2) interposed between one face of the support sheet and the layer (3) of particles in order to hold the latter on the support sheet (1), this substrative layer (2) consisting of a substance which is miscible with the solution of active principle.

2. Reservoir according to Claim 1, characterized in that the thickness of the said layer (3) of particles is of the order of magnitude of the mean size of these particles.

3. Reservoir according to either of Claims 1 or 2, characterized in that it is lined with a double-sided adhesive sheet, bonded to the support sheet (1) on that face of the latter which is opposite the face coated with the substrative layer (2).

4. Reservoir according to Claim 3, characterized in that the said adhesive sheet is bonded by its other face onto a metal layer constituting an electrode.

5. Reservoir according to either of Claims 3 or 4, characterized in that a sheet (6) of an open-worked material is interposed between the support sheet (1) and the double-sided adhesive sheet.

6. Reservoir according to any one of Claims 1 to 5, characterized in that the support sheet (1) made of cavitied material is impregnated with a polyoxyethylene hydrogel prior to depositing of the substrative layer.

7. Reservoir according to any one of Claims 1 to 6, characterized in that it comprises a cover sheet (5) made of a cavitied material, covering the layer (3) of particles of the impregnatable material.

8. Reservoir according to any one of Claims 1 to 7, characterized in that the various sheets and layers forming it conform and are welded to one another by the edges.

9. Reservoir according to any one of Claims 1 to 8, characterized in that the support sheet (1) is made of one of the products in the group consisting of woven or non-woven products, a felt, a mesh, a cavitied foam, made of a polymer component in the group consisting of polyethylene, polypropylene, polyamides, polyesters, polyurethane, cellulose, viscose, olefinic copolymers, polyvinyl chloride, cellulosic, acrylic or methacrylic polymers.

10. Reservoir according to any one of Claims 1 to 9, characterized in that the substrative layer (2) consists of one of the substances in the group consisting of: glycerol, diacetin, homoacetin, polyethylene glycol.

11. Reservoir according to any one of Claims 1 to 10, characterized in that the impregnatable divided material consists of one of the substances in the group formed by: cellulosic derivatives such as hydroxypropylmethylcellulose, hydroxyethylcellulose, hydroxypropylcellulose, carboxymethylcellulose, methylcellulose, polymers and copolymers of acrylic acid, of methacrylic acid and of their esters, polyalkylene glycol gels, polyvinylpyrrolidone and its derivatives, polyvinyl alcohol, polyacrylamide and derivatives, natural gums, hydrosoluble amylaceous derivatives and copolymers (methylvinylether/maleic-anhydride).

12. Reservoir according to Claim 11, characterized in that the impregnatable divided material consists of 80 µm to 250 µm particles, the layer (3) of this material deposited on the substrative layer (2) having a maximum thickness of approximately 300 µm.

13. Reservoir according to both of Claims 7 and 9, characterized in that the cover sheet (5) and the support sheet (1) consist of polymer substances of the same group.

14. Reservoir according to any one of Claims 5 to 13, characterized in that the sheet (6) of open-worked material consists of a mesh, a felt, a woven or non-woven product made of polymer material.

15. Method of manufacture of a reservoir according to any one of Claims 1 to 14, characterized in that (a) a face of the support sheet (1) is coated with an adhesive layer (2) of a substrative product and (b) the layer thus formed is powdered with particles (4ᵢ) of the material which can be impregnated with the solution of active principle.

16. Method according to Claim 15, characterized in that the support sheet (1) takes the form of a band moving through a station (7, 8) for coating one face of this band with the substrative substance, then through a station (9, 10, 11) for powdering the layer of this substance formed on the band.

17. Method according to Claim 16, characterized in that the moving band is lined with a band (13) of a peel-off protective material.

18. Method according to Claim 17, characterized in that the lined band is impregnated with a polyoxyethylene hydrogel, then dried, before passing through the station (7, 8) for coating with the substrative substance.

19. Method according to either of Claims 17 or 18, characterized in that, at the exit of the powdering station (9, 10, 11), the protective band (13) is peeled off and the substrate-coated and powdered band (1, 2, 3) is then pressed between a cover band (5) made of an open-worked material covering the layer (3) of powder and a mesh band (6) applied against the other face of the substrate-coated and powdered band (1, 2, 3).

20. Method according to Claim 19, characterized in that the band product obtained is evenly welded to the final dimensions of a reservoir then cut out along these welds (16, 17) in order to separate each reservoir.

21. Method according to Claim 19, characterized in that the band product obtained is hot-calendered in order to reinforce its cohesion.

22. Method according to any one of Claims 15 to 21, characterized in that the particles of the impregnatable material are sieved in order to form a layer of such particles on the adhesive substrative layer (2).

## Patentansprüche

1. Mit einer Wirkstofflösung tränkbarer Behälter zur Verwendung in einer Iontophoresevorrichtung zur transdermalen Verabreichung von Arzneimitteln, wobei dieser Behälter mit einem mit dieser Lösung tränkbaren, zerteilten Material beladen ist, dadurch gekennzeichnet, daß er folgendes aufweist:
a) eine Tragfolie (1) aus einem mit Lücken versehenen Material, die für die Lösung durchlässig ist und zur mechanischen Festigkeit des Behälters beiträgt,
b) eine Schicht (3) von Teilchen aus dem tränkbaren Material und
c) eine Substratbildungsschicht (2), die zwischen eine Seite der Tragfolie und die Schicht (3) von Teilchen eingesetzt ist, um diese auf der Tragfolie (1) zurückzuhalten, wobei diese Substratbildungsschicht (2) aus einer mit der Wirkstofflösung mischbaren Substanz besteht.

2. Behälter nach Anspruch 1, dadurch gekennzeichnet, daß die Dicke der Teilchenschicht (3) von der Größenordnung der mittleren Abmessung dieser Teilchen ist.

3. Behälter nach einem der Ansprüche 1 und 2, dadurch gekennzeichnet, daß er mit einer doppelseitigen Klebefolie belegt ist, die an die Tragfolie (1) an deren Seite angefügt ist, die der mit der Substratbildungsschicht (2) beschichteten Seite entgegengesetzt ist.

4. Behälter nach Anspruch 3, dadurch gekennzeichnet, daß die Klebefolie mit ihrer anderen Seite an eine eine Elektrode bildende Metallschicht angeklebt ist.

5. Behälter nach einem der Ansprüche 3 und 4, dadurch gekennzeichnet, daß eine Folie (6) aus einem durchbrochenen Material zwischen die Tragfolie (1) und die doppelseitige Klebefolie eingesetzt ist.

6. Behälter nach einem der Ansprüche 1 bis 5, dadurch gekennzeichnet, daß die Tragfolie (1) aus einem Lücken aufweisenden Material vor der Aufbringung der Substratbildungsschicht mit einem Polyoxyethylenhydrogel getränkt wird.

7. Behälter nach einem der Ansprüche 1 bis 6, dadurch gekennzeichnet, daß er eine Deckfolie (5) aus einem Lücken aufweisenden Material besitzt, die die Schicht (3) von Teilchen aus dem tränkbaren Material bedeckt.

8. Behälter nach einem Ansprüche 1 bis 7, dadurch gekennzeichnet, daß die einzelnen ihn bildenden Folien und Schichten geformt sind und an Rändern miteinander verschweißt sind.

9. Behälter nach einem der Ansprüche 1 bis 8, dadurch gekennzeichnet, daß die Tragfolie (1) aus einem der Produkte der Gruppe hergestellt ist, die besteht aus: gewebte oder nicht gewebte Produkte, Filz, Gitter, poröser Schaumstoff, eine Polymerverbindung der Gruppe, die besteht aus Polyethylen, Polypropylen, den Polyamiden, den Polyestern, Polyurethan, Cellulose, Viskose, den Olefincopolymeren, Polyvinylchlorid, den Cellulose-, Acryl- oder Methacrylpolymeren.

10. Behälter nach einem der Ansprüche 1 bis 9, dadurch gekennzeichnet, daß die Substratbildungsschicht (2) aus einer der Substanzen der Gruppe gebildet ist, die besteht aus: Glycerin, Diacetin, Homoacetin, Polyethylenglykol.

11. Behälter nach einem Ansprüche 1 bis 10, dadurch gekennzeichnet, daß das tränkbare, zerteilte Material von einer der Substanzen der Gruppe gebildet ist, die besteht aus: Cellulosederivate wie Hydroxypropyl-methylcellulose, Hydroxyethylcellulose, Hydroxypropylcellulose, Carboxymethylcellulose, Methylcellulose, die Polymere und Copolymere der Acrylsäure, der Methacrylsäure und deren Ester, die Polyalkylenglykolgele, Polyvinylpyrrolidon und seine Derivate, Polyvinylalkohol, Polyacrylamid und Derivate, die natürlichen Gummen, die wasserlöslichen Stärkederivate und die Copolymere (Vinyl Methyl Ether-Malleinsäureanhydrid).

12. Behälter nach Anspruch 11, dadurch gekennzeichnet, daß das tränkbare, zerteilte Material aus Teilchen von 80 µm bis 250µm besteht, wobei die auf die Substratbildungsschicht (2) aufgebrachte Schicht (3) aus diesem Material eine Dicke von höchstens etwa 300 µm aufweist.

13. Behälter nach den Ansprüchen 7 und 9 zusammen, dadurch gekennzeichnet, daß die Deckfolie (5) und die Tragfolie (1) aus Polymersubstanzen derselben Gruppe gebildet sind.

14. Behälter nach einem der Ansprüche 5 bis 13, dadurch gekennzeichnet, daß die Folie (6) aus durchbrochenem Material aus einem Gitter, einem Filz, einem gewebten oder nicht gewebten Produkt, aus Polymermaterial besteht.

15. Verfahren zur Herstellung eines Behälters nach einem der Ansprüche 1 bis 14, dadurch gekennzeichnet, daß man (a) eine Seite der Tragfolie (1) mit einer Klebeschicht (2) aus einem Substratbildungsprodukt beschichtet und b) die so gebildete Schicht mit Teilchen (4ᵢ) des mit der Wirkstofflösung tränkbaren Materials bestäubt.

16. Verfahren nach Anspruch 15, dadurch gekennzeichnet, daß die Tragfolie (1) die Form eines Bandes erhält, das durch eine Vorrichtung (7, 8) zur Beschichtung einer Seite dieses Bandes mit der Substratbildungssubstanz und dann durch eine Vorrichtung (9, 10, 11) zum Bestäuben der auf dem Band gebildeten Schicht aus dieser Substanz läuft.

17. Verfahren nach Anspruch 16, dadurch gekennzeichnet, daß auf das durchlaufende Band ein abziehbares Band (13) aus einem Schutzmaterial aufgelegt wird.

18. Verfahren nach Anspruch 17, dadurch gekennzeichnet, daß das aufgelegte Band mit einem Polyoxyethylenhydrogel getränkt und dann getrocknet wird, bevor es in die Vorrichtung (7, 8) zur Beschichtung mit der Substratbildungsschicht eintritt.

19. Verfahren nach einem der Ansprüche 17 und 18, dadurch gekennzeichnet, daß das Schutzband (13) am Austritt aus der Bestäubungsvorrichtung (9, 10, 11) abgezogen wird und das mit Substrat versehene und bestäubte Band (1, 2, 3) anschließend zwischen einem die Pulverschicht (3) bedeckenden Deckband (5) aus einem durchbrochenen Material und einem Gitterband (6) eingeklemmt wird, das auf die andere Seite des mit Substrat versehenen und bestäubten Bandes (1, 2, 3) aufgebracht wird.

20. Verfahren nach Anspruch 19, dadurch gekennzeichnet, daß das erhaltene Bandprodukt regelmäßig in den Endabmessungen eines Behälters verschweißt wird und dann längs dieser Verschweißungen (16, 17) zur Abtrennung jedes Behälters geschnitten wird.

21. Verfahren nach Anspruch 19, dadurch gekennzeichnet, daß das erhaltene Bandprodukt zur Verstärkung seiner Kohäsion heiß kalandriert wird.

22. Verfahren nach einem der Ansprüche 15 bis 21, dadurch gekennzeichnet, daß man die Teilchen des tränkbaren Materials siebt, um eine Schicht aus solchen Teilchen auf der Substratbildungsklebeschicht (2) zu bilden.
